# EUROPÄISCHE PATENTSCHRIFT

(11) **EP 0 688 764 B1**
(45) Veröffentlichungstag und Bekanntmachung des Hinweises auf die Patenterteilung: **10.02.1999**
(21) Anmeldenummer: 95109311.1
(22) Anmeldetag: 16.06.1995
(51) Int. Cl.: C07C 249/04, C07C 251/44

(54) **Verfahren zur Herstellung von aliphatischen und cycloaliphatischen Oximen**
Process for the preparation of aliphatic and cycloaliphatic oximes
Procédé pour la préparation d'oximes aliphatiques et cycloaliphatiques

(30) Priorität: 23.06.1994 DE 4421928
(43) Veröffentlichungstag der Anmeldung: 27.12.1995
(73) Patentinhaber: BASF Aktiengesellschaft, 67063 Ludwigshafen (DE)
(72) Erfinder: Rieber, Norbert, Dr., D-68259 Mannheim (DE); Lingelbach, Peter, Dr., D-67063 Ludwigshafen (DE); Witzel, Tom, Dr., D-67069 Ludwigshafen (DE); Müller, Ulrich, Dr., D-67434 Neustadt (DE)

(56) Entgegenhaltungen:
- EP-A- 0 043 445
- EP-A- 0 267 362
- EP-A- 0 299 430
- EP-A- 0 301 486
- EP-A- 0 314 582
- EP-A- 0 337 835
- EP-A- 0 347 926
- EP-A- 0 384 390
- EP-A- 0 395 046
- DE-A- 3 047 798
- US-A- 4 504 681
- CHEMICAL ABSTRACTS, vol. 116, no. 2, 1992, Columbus, Ohio, US; abstract no. 8204d, & J. MOL. CATAL., Bd.69, Nr.2, 1991 Seiten 171 - 190 D.P. DREONI

## Beschreibung

Die vorliegende Erfindung betrifft ein Verfahren zur Herstellung von aliphatischen oder cycloaliphatischen Oximen.

Cyclohexanonoxim, bekanntermaßen ein wichtiges Zwischenprodukt bei der Herstellung von Caprolactam, wird üblicherweise durch Umsetzung von Cyclohexanon mit Hydroxylaminsalzen hergestellt. Die dabei in großen Mengen anfallenden Salze kann man beispielsweise durch das Hydroxylamin-Phosphat-Oxim-(HPO)-Verfahren (siehe Weissermel, Arpe, 3. Auflage, VCH-Verlag, 1988, S. 270 ff.), bei dem man phosphorsäurehaltige Puffer einsetzt, vermeiden. Die Herstellung des nur mäßig stabilen Hydroxylamins wird dabei jedoch nicht umgangen.

Nach der US 4,163,756 kann man Cyclohexanonoxim auch durch Direktoximierung mit einem Ammoniak/Luft-Gemisch in der Gasphase mit einer Selektivität für das Oxim zu 51 % bei einem Umsatz von 54 % herstellen. Nachteilig an dieser Verfahrensweise ist jedoch, daß für eine wirtschaftliche Nutzung die Selektivitäten und Umsätze zu niedrig sind. Des weiteren ist durch Polymerbildung die katalytische Aktivität des verwendeten Katalysators zu schnell erschöpft.

Nach der EP-A 208,311 wird Cyclohexanon in der Flüssigphase mit Wasserstoffperoxid und Ammoniak zum entsprechenden Oxim umgesetzt, wobei die Selektivität 80 % bei einem Umsatz von 95 % beträgt. Der Einsatz von Wasserstoffperoxid ist jedoch aus Kostengründen wirtschaftlich ungünstig. Zudem ist aus Gründen der Sicherheit der Einsatz von Wasserstoffperoxid nachteilig.

Die EP-A 395,046 beschreibt die Oxidation von Cyclohexylamin durch Direktoxidation in der Flüssigphase mit homogenen und heterogenen titanhaltigen Katalysatoren. Die erreichten Selektivitäten betragen bei Umsätzen von 60 % bis zu 52 %, womit die Ausbeuten maximal nur 31,2 % betragen. Zudem ist diese Verfahrensweise wegen des relativ teuren Einsatzstoffes Cyclohexylamin auch aus wirtschaftlichen Gründen nicht von Vorteil.

Der vorliegenden Erfindung lag daher die Aufgabe zugrunde, ein Verfahren zur Herstellung von aliphatischen oder cycloaliphatischen Oximen zur Verfügung zu stellen, das die obengenannten Nachteile nicht aufweist. Insbesondere sollte ein wirtschaftliches Verfahren unter Vermeidung der bekannten Salzabfälle gefunden werden.

Demgemäß wurde ein Verfahren zur Herstellung von aliphatischen oder cycloaliphatischen Oximen gefunden, indem man aliphatische oder cycloaliphatische Imine mit Sauerstoff oder einem sauerstoffhaltigen Gas in Gegenwart eines Katalysators behandelt.

Erfindungsgemäß setzt man die aliphatischen oder cycloaliphatischen Imine mit Sauerstoff oder einem sauerstoffhaltigen Gas in Gegenwart eines Katalysators zum entsprechenden Oxim um.

Als aliphatische und cycloaliphatische Imine setzt man im allgemeinen Imine der allgemeinen Formel I

R¹R²C=NH (II)

ein, in der R¹ C₁-C₁₀-Alkyl wie Methyl, Ethyl, n-, i-Propyl, n-, sek.-, i-, tert.-Butyl, n-Pentyl, n-Hexyl, n-Heptyl, n-Octyl, n-Nonyl und n-Decyl, und R² Wasserstoff und C₁-C₁₀-Alkyl wie Methyl, Ethyl, n-, i-Propyl, n-, sek.-, i-, tert.Butyl, n-Pentyl, n-Hexyl, n-Heptyl, n-Octyl, n-Nonyl und n-Decyl, bedeuten, oder R¹ und R² zusammen C₃-C₁₂-Cycloalkyliden wie Cyclopropyliden, Cyclobutyliden, Cyclopentyliden, Cyclohexyliden, Cycloheptyliden, Cyclooctyliden, Cyclononyliden, Cyclodecyliden, Cycloundecyliden, Cyclododecyliden, bedeuten.

Als bevorzugte Imine seien genannt: Cyclopentylidenamin, Cyclo-hexylidenamin ("Cyclohexanonimin") und Cycloheptylidenamin, besonders bevorzugt Cyclohexylidenamin ("cyclohexanonimin").

Die Imine sind nach literaturbekannten Verfahren zugänglich, die beispielsweise in Houben-Weyl, Band 7, Teil 2b, Keton II, S. 1947 (4. Auflage 1976, Georg Thieme Verlag Stuttgart) beschrieben sind.

In einer bevorzugten Ausführungsform (analog zu dem Verfahren aus der EP-A 449 111) stellt man die cycloaliphatischen Imine durch Iminierung der entsprechenden cycloaliphatischen Ketone her. Hierbei werden im allgemeinen die entsprechenden Cycloalkanone mit überschüssigem Ammoniak bei Temperaturen im Bereich von 20 bis 150°C, vorzugsweise von 30 bis 130°C, besonders bevorzugt von 50 bis 100°C und Drücken im Bereich von 15 bis 500 bar, vorzugsweise von 100 bis 350 bar zu den entsprechenden Cycloalkyliden-aminen in Gegenwart von aciden Heterogenkatalysatoren umgesetzt.

Als acide Heterogenkatalysatoren eignen sich Metallverbindungen mit Lewissäure- oder Brönstedtsäure-Charakter wie Aluminiumoxid, Siliciumdioxid, Titandioxid, Zirkondioxid, ferner Phosphate, wie Aluminiumphosphate oder Silikate wie amorphe oder kristalline Aluminosilikate. Bevorzugt verwendet man Aluminiumoxid, Titandioxid, Zirkondioxid und Siliciumdioxid, insbesondere Aluminiumoxid und Titandioxid. Die Acidität der Katalysatoren kann gegebenenfalls durch Dotierung mit Halogeniden erhöht werden. So finden beispielsweise auch halogendotierte Katalysatoren wie Chlorid auf Aluminiumoxid oder Chlorid auf Titandioxid Verwendung.

Bei der bevorzugten Iminierung hält in der Regel man eine Katalysatorbelastung von 0,01 bis 10, vorzugsweise von 0,05 bis 7, besonders bevorzugt von 0,1 bis 5 kg Cycloalkanon pro kg Katalysator und Stunde ein.

Die Menge an Ammoniak wählt man üblicherweise im Bereich von 5 bis 500, bevorzugt von 10 bis 400, besonders bevorzugt von 20 bis 300 Mol Ammoniak pro Mol Cycloalkanon.

Die Verwendung eines Lösungsmittels wie Alkanole oder Tetrahydrofuran ist optional.

Des weiteren kann man die bevorzugte Iminierung sowohl kontinuierlich als auch diskontinuierlich durchführen, bevorzugt kontinuierlich beispielsweise in Druckbehältern oder Druckbehälterkaskaden. Nach einer besonders bevorzugten Ausführungsform leitet man die Cycloalkanone und Ammoniak durch einen Rohrreaktor, in dem der Iminisierungskatalysator in Form eines festen Bettes angeordnet ist.

Die Reaktionszeit bei der Iminierung hängt im wesentlichen von der Katalysatorbelastung und der Menge an eingesetztem Ammoniak ab. Sie liegt in der Regel im Bereich von 0,5 bis 120 Minuten, bevorzugt von 1 bis 40 Minuten, besonders bevorzugt von 1,5 bis 20 Minuten.

Bei der Umsetzung der Imine mit Sauerstoff zu den entsprechenden Oximen wählt man in der Regel ein Molverhältnis von Imin zu Sauerstoff im Bereich von 0,1 bis 100, bevorzugt von 1 bis 10. Als sauerstoffhaltiges Gas verwendet man üblicherweise Luft, wobei man in der Regel das gleiche Molverhältnis von Imin zu Sauerstoff wählt. Man kann auch andere sauerstoffhaltigen Gasgemische einsetzen, sofern sich die nicht-sauerstoffhaltigen Gase gegenüber den anderen Reaktionsteilnehmern inert verhalten.

Man setzt Katalysatoren ausgewählt aus der Gruppe, bestehend aus Ti(OR)₄, Zr(OR)₄, Hf(OR)₄, wobei R für C₁-C₁₀-Alkyl steht, ein. Bevorzugte Katalysatoren sind Ti(OEt)₄, Ti(O-iPr)₄, Ti(OBu)₄, Zr(OEt)₄, Zr(O-iPr)₄, Zr(OBu)₄, besonders bevorzugt Ti(OEt)₄, Ti(O-iPr)₄, Ti(OBu)₄ und Zr(OEt)₄.

Die Menge an Katalysator, bezogen auf eingesetztes Imin, wählt man üblicherweise im Bereich von 0,001 bis 10, bevorzugt von 0,1 bis 2 Gew.-%.

Die Oximierung kann man sowohl mit als auch ohne lösungsmittel durchführen. Bevorzugt führt man die Reaktion in einem gegenüber Sauerstoff inerten Lösungsmittel durch. Beispielhaft seien genannt aromatische Kohlenwasserstoffe, die gewünschtenfalls halogeniert sein können, wie Chlorbenzol, Benzol, Dichlorbenzol, Toluol, Xylol, bevorzugt chlorbezzol.

Das Gewichtsverhältnis von Lösungsmittel zu Imin wählt man in der Regel im Bereich von 50:1 bis 1:1, vorzugsweise von 10:1 bis 2:1.

In einer weiteren bevorzugten Ausführungsform führt man die Oximierung des Imins in Gegenwart des entsprechenden Ketons durch. So kann man beispielsweise die Oximierung von Cyclohexylidenamin in Gegenwart von Cyclohexanon durchführen. Diese Verfahrensweise ist insofern vorteilhaft, weil bei der Herstellung des Imins aus dem entsprechenden Keton das Keton nicht unbedingt vor der Oximierung abgetrennt werden muß.

Die Reaktionstemperatur wählt man im allgemeinen im Bereich von 20 bis 200, bevorzugt von 50 bis 150°C.

Den Druck während der Reaktion wählt man in der Regel im Bereich von 100 bis 50000, bevorzugt von 3000 bis 10000 kPa.

Üblicherweise führt man die Reaktion in flüssiger Phase durch. Prinzipiell kann man die Reaktion auch in gasförmiger oder flüssig/gasförmiger Phase durchführen.

Die Reaktionsdauer hängt naturgemäß im wesentlichen von den gewählten Reaktionsparametern ab und liegt üblicherweise bei diskontinuierlicher Fahrweise im Bereich von 1 bis 100, bevorzugt von 5 bis 20 h.

Nach der Oximierung arbeitet man üblicherweise das Reaktionsgemisch nach an sich bekannten Methoden wie Destillation oder Extraktion auf.

In einer weiteren bevorzugten Ausführungsform stellt man die aliphatischen oder cycloaliphatischen Oxime zweckmäßig so her, daß man zunächst das Imin aus dem entsprechenden Keton herstellt, und im Anschluß daran das das Imin enthaltende Reaktionsgemisch der Oximierung unterwirft, d.h. man führt folgende Schritte durch:
a) Iminierung eines aliphatischen oder cycloaliphatischen Ketons und
b) Oxidation des in Stufe a) erhaltenen Imins zum entsprechenden Oxim.

Diese bevorzugte Verfahrensweise hat den Vorteil, daß das zu oximierende Imin nach den bisherigen Beobachtungen weitgehend von störenden, durch Abbau des Imins erhaltenen Nebenprodukten frei ist.

Das nach dem erfindungsgemäßen Verfahren erhältliche Cyclo-hexanonoxim läßt sich nach bekannten Verfahren (siehe beispielsweise Weissermel, Arpe, 3. Auflage, VCH-Verlag, 1988, S.270 ff) zu Caprolactam umsetzen.

Das erfindungsgemäße Verfahrens weist gegenüber den Verfahren des Standes der Technik die Vorteile auf, daß man Salzabfälle sowie teure und sicherheitsbedenkliche Ausgangsstoffe vermeidet und als Oxidationsmittel kostenlos zur Verfügung stehende Luft einsetzen kann.

### Beispiele

Die Charakterisierung der Stoffe sowie die Bestimmungen der Umsätze und Selektivitäten wurden gaschromatographisch durchgeführt.

### Beispiel 1 bis 6 und Vergleichsbeispiele 7 bis 9

10 g Cyclohexylidenamin (Cyclohexanonimin), 100 ml Chlorbenzol und 1 g Tetrakis(n-butoxy)titanat wurden in einem 300-ml-Rührautoklaven mit Luft versetzt, wobei der Sauerstoffpartialdruck 5000 kPa betrug, und auf 100°C erhitzt. Nach 20 h wurde die Reaktion abgebrochen. Die Selektivität betrug bei einem Umsatz von 100 %, bezogen auf eingesetztes Imin, 46 %.

Analog zu Beispiel 1 wurden die in nachfolgender Tabelle angegebenen Ausgangsverbindungen in den dort angegebenen Mengen und Reaktionsbedingungen zu den entsprechenden Oximen umgesetzt. Die berechneten Selektivitäten finden sich ebenfalls in dieser Tabelle.

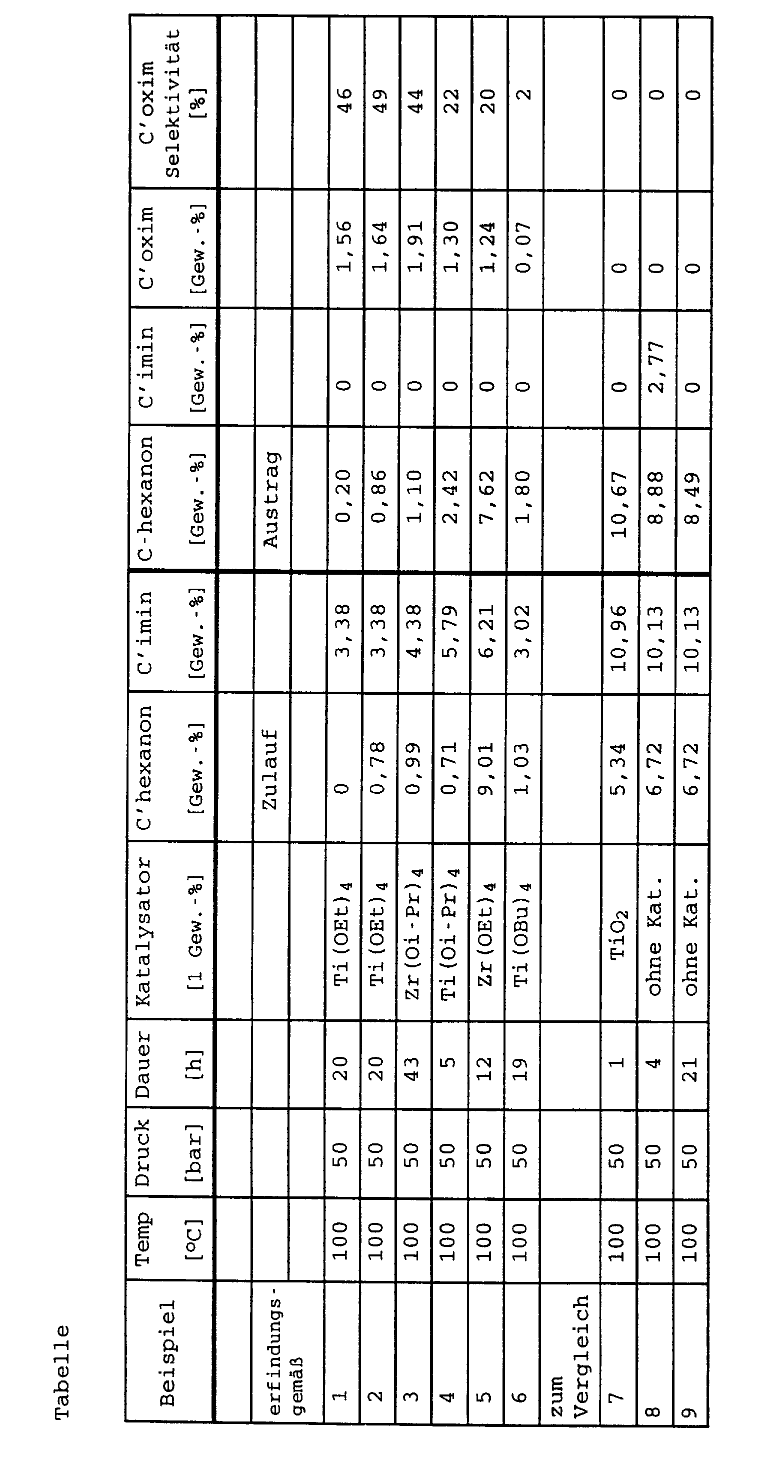

## Patentansprüche

1. Verfahren zur Herstellung von aliphatischen und cycloaliphatischen Oximen, dadurch gekennzeichnet, daß an aliphatische oder cycloaliphatische Imine mit Sauerstoff oder einem sauerstoffhaltigen Gas in Gegenwart eines Katalysators, wobei man Katalysatoren einsetzt, ausgewählt aus der Gruppe, bestehend aus Ti(OR)₄, Zr(OR)₄, Hf(OR)₄, wobei R für C₁-C₁₀-Alkyl steht, behandelt.

2. Verfahren nach Anspruch 1, dadurch gekennzeichnet, daß man C₃-C₁₂-Cycloalkylidenamine einsetzt.

3. Verfahren nach Anspruch 2, dadurch gekennzeichnet, daß man als Cycloalkylidenamin Cyclohexylidenamin einsetzt.

4. Verfahren gemäß den Ansprüchen 1 bis 3, dadurch gekennzeichnet, daß man die Reaktion in Gegenwart des entsprechenden Ketons durchführt.

## Claims

1. A process for the preparation of an aliphatic or cycloaliphatic oxime, wherein an aliphatic or cycloaliphatic imine is treated with oxygen or with an oxygen-containing gas in the presence of a catalyst, the catalyst used being selected from the group consisting of Ti(OR)₄, Zr(OR)₄ and Hf(OR)₄, where R is C₁-C₁₀-alkyl.

2. A process as claimed in claim 1, wherein a C₃-C₁₂-cycloalkylideneamine is used.

3. A process as claimed in claim 2, wherein the cycloalkylideneamine used is cyclohexylideneamine.

4. A process as claimed in any of claims 1 to 3, wherein the reaction is carried out in the presence of the corresponding ketone.

## Revendications

1. Procédé de préparation d'oximes aliphatiques et cycloaliphatiques caractérisé en ce que l'on traite des imines aliphatiques ou cycloaliphatiques avec de l'oxygène ou avec un gaz contenant de l'oxygène, en présence d'un catalyseur, les catalyseurs utilisés étant choisis dans le groupe formé par Ti(OR)₄, Zr(OR)₄, Hf(OR)₄, R représentant un groupement alkyle en C₁-C₁₀.

2. Procédé selon la revendication 1, caractérisé en ce que l'on utilise des cycloalkylidène-amines en C₃-C₁₂.

3. Procédé selon la revendication 2, caractérisé en ce que l'on utilise des cyclohexylidène-amines en tant que cycloalkylidène-amines.

4. Procédé selon l'une quelconque des revendications 1 à 3, caractérisé en ce que l'on effectue la réaction en présence de la cétone correspondante.
